# EUROPEAN PATENT APPLICATION

(11) **EP 4 190 315 A1**
(43) Date of publication of application: **07.06.2023**
(21) Application number: 21864641.2
(22) Date of filing: 01.09.2021
(51) Int. Cl.: A61K 9/70, A61K 47/36, A61K 47/42, A61K 47/10, A61K 47/18, A61K 47/12

(54) **EXOSOMES-LOADED BIOMIMETIC TISSUE-ADHESIVE HYDROGEL PATCH**

(30) Priority: 01.09.2020 KR 20200111337
(71) Applicant: Cellartgen Inc., Seoul 03722 (KR)
(72) Inventor: CHO, Seung Woo, Seoul 03722 (KR); HAN, Seung Yeop, Seoul 03722 (KR); JEON, Eun Je, Seoul 03722 (KR)
(74) Representative: Manitz Finsterwald Patent- und Rechtsanwaltspartnerschaft mbB
(86) International application number: PCT/KR2021/011755
(87) International publication number: WO 2022/050682

(57) **Abstract**

The present disclosure relates to a hydrogel patch, including: a hydrogel patch containing a biocompatible polymer functionalized with a phenol group; and an exosome loaded in the hydrogel patch.

## Description

### TECHNICAL FIELD

The present disclosure relates to an exosome-loaded bio-inspired tissue-adhesive hydrogel patch.

### BACKGROUND

The Drug controlled release technology market is expected to grow from $28.5 billion in 2016 to 2025, at a continuous compound annual growth rate of 13.8%. However, in many cases, it is necessary to enhance drug delivery efficiency, and the types and indications of deliverable drugs are limited. Therefore, a drug delivery system capable of loading various drugs and enabling more efficient sustained release is a high value-added medical technology and can create a great ripple effect in terms of health and society.

Meanwhile, cell therapeutic agents using various stem cells are under intense research for treatment of intractable diseases and tissue regeneration. However, stem cells are difficult to apply clinically and commercialize due to side effects, such as cancer induction and immune response, and safety issues. Therefore, recently, a strategy to apply factors secreted from stem cells as therapeutic agents instead of cells in order to substitute for stem cell therapy has received lots of attention. In particular, exosomes secreted by stem cells are known to contain various factors that promote tissue regeneration and induce anti-inflammatory action. Thus, exosomes extracted and separated from stem cells are under intense research for application to various intractable diseases, such as wounds, myocardial infarctions, cerebral infarctions, spinal cord injury, inflammatory bowel diseases and various ischemic diseases.

Exosomes extracted from stem cells substitute for stem cell therapeutic agents, which are difficult to apply clinically due to safety issues, and many studies have been reported showing excellent efficacy in treatment of various diseases such as diabetic wounds, myocardial infarctions, cerebral infarctions, spinal cord injury, inflammatory bowel diseases, various ischemic diseases and hair loss. The market size of exosomes as therapeutic agents is expected to grow at a compound annual growth rate of 37.8% and to reach $368 million (USD) by 2022. Accordingly, the development of a delivery system that can naturally maximize the efficiency of exosome delivery into the body will be required. Therefore, the hydrogel patch-based exosome delivery technology developed in the present disclosure is expected to create an enormously high added value in view of the huge market size of various diseases to which exosome-based treatment can be applied.

However, despite the excellent regenerative therapeutic efficacy of exosomes, a technology for efficiently delivering exosomes into the body is underdeveloped. Drug delivery systems based on biomaterials and nanomaterials have been developed to deliver protein growth factors and anti-inflammatory drugs, but such conventional systems have not been applied to many cases of exosome delivery. In particular, hydrogel-based topical drug delivery systems have been developed, but conventional liquid hydrogels need to be improved in terms of biocompatibility, mechanical properties, tissue adhesion and ease of use and further improved in terms of sustained delivery efficiency.

Therefore, in the present disclosure, a patch-type hydrogel that overcomes the limitations of the conventional drug delivery technology and is capable of sustained delivery of exosomes is fabricated. Exosomes extracted from human adipose-derived stem cells are loaded in a phenol group-functionalized hyaluronic acid derivative-based hydrogel patch, and, thus, the hydrogel patch is significantly improved in terms of tissue adhesion, mechanical properties and sustained exosome release ability compared with the conventional liquid hydrogels.

### DISCLOSURE OF THE INVENTION

### PROBLEMS TO BE SOLVED BY THE INVENTION

The present disclosure is intended to provide an exosome-loaded bio-inspired tissue-adhesive hydrogel patch with excellent mechanical properties, tissue adhesion, biocompatibility and ease of use, and provides an exosome hydrogel patch, including: a hydrogel patch containing a biocompatible polymer functionalized with a phenol group; and an exosome loaded in the hydrogel patch.

### MEANS FOR SOLVING THE PROBLEMS

An aspect of the present disclosure provides an exosome hydrogel patch, including: a hydrogel patch containing a biocompatible polymer functionalized with a phenol group; and an exosome loaded in the hydrogel patch.

In an embodiment of the present disclosure, the phenol group may be a catechol group derived from a catechol-based compound selected from the group consisting of catechol, 4-tert-butylcatechol (TBC), urushiol, alizarin, dopamine, dopamine hydrochloride, 3,4-dihydroxyphenylalanine (DOPA), caffeic acid, norepinephrine, epinephrine, 3,4-dihydroxyphenylacetic acid (DOPAC), isoprenaline, isoproterenol and 3,4-dihydroxybenzoic acid; or a pyrogallol group derived from a pyrogallol-based compound selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol.

In an embodiment of the present disclosure, the biocompatible polymer may be selected from the group consisting of hyaluronic acid, heparin, cellulose, dextran, alginate, chitosan, chitin, collagen, gelatin, chondroitin sulfate, pectin, keratin and fibrin.

In an embodiment of the present disclosure, the exosome hydrogel patch may have i) a thickness of from 0.05 mm to 10.0 mm, ii) a storage modulus G' of from 1×10³ Pa to 1×10⁶ Pa and tanδ of from 0.01 to 0.15 in a frequency range of from 0.1 Hz to 10 Hz, iii) a friction factor of from 0.2 to 0.4 as measured at a speed of 0.01 m/s under a normal force of 5 N, and iv) an adhesive strength of from 0.1 N to 10 N.

In an embodiment of the present disclosure, the exosome may be derived from stem cells.

### EFFECTS OF THE INVENTION

An exosome-loaded phenol group-functionalized hyaluronic acid hydrogel patch according to the present disclosure can be fabricated in ready-to-use and off-the-shelf formulations which are freeze-dried with exosomes loaded and thus highly enhances user convenience and can be easily applied to various disease sites by medical staff. Also, the hydrogel patch is expected to be loaded with various vesicles secreted by cells as well as exosomes and to deliver the same in an efficient manner for use in treatment. A treatment technology based on factors secreted from cells can induce cell therapeutic effect without cell engraftment and thus can be rapidly developed and is more preferable in terms of safety. Therefore, it is highly likely to be commercialized. Accordingly, it has a great potential as a technology for treating intractable diseases to substitute for a significant portion of the huge stem cell therapeutic agent market.

### BRIEF DESCRIPTION OF THE DRAWINGS

**FIG. 1A** shows the structural formula of HA-CA.
**FIG. 1B** shows the result of analyzing a swelling ratio of an HA-CA hydrogel patch.
**FIG. 1C** shows the result of analyzing a degradation rate of the HA-CA hydrogel patch by an enzyme.
**FIG. 2A** shows the result of analyzing a storage modulus and a loss modulus of the HA-CA hydrogel patch.
**FIG. 2B** shows the result of analyzing an average storage modulus of the HA-CA hydrogel patch.
**FIG. 3A** shows the result of analyzing a friction factor of the HA-CA hydrogel patch.
**FIG. 3B** shows the result of analyzing the degree of wear scar of the HA-CA hydrogel patch.
**FIG. 3C** shows the result of analyzing the area of wear scar of the HA-CA hydrogel patch.
**FIG. 4A** shows the structural formula of HA-PG.
**FIG. 4B** shows the result of analyzing a swelling ratio of the HA-PG hydrogel patch.
**FIG. 4C** shows the result of analyzing a degradation rate of the HA-PG hydrogel patch by an enzyme.
**FIG. 5A** shows the result of analyzing a storage modulus and a loss modulus of the HA-PG hydrogel patch.
**FIG. 5B** shows the result of analyzing an average storage modulus of the HA-PG hydrogel patch.
**FIG. 6A** shows the result of analyzing a friction factor of the HA-PG hydrogel patch.
**FIG. 6B** shows the result of analyzing the area of wear scar of the HA-PG hydrogel patch.
**FIG. 6C** shows the result of analyzing the degree of wear scar of the HA-PG hydrogel patch.
**FIG. 7** shows a method of fabricating an exosome-loaded HA-PG hydrogel.
**FIG. 8a** shows the result of analyzing an exosome extracted from human adipose-derived mesenchymal stem cells and the result of analyzing a structural change inside the HA-PG hydrogel when the exosome is introduced into the HA-PG hydrogel patch.
**FIG. 8b** shows the result of analyzing an exosome extracted from human adipose-derived mesenchymal stem cells and the result of analyzing a structural change inside the HA-PG hydrogel when the exosome is introduced into the HA-PG hydrogel patch.
**FIG. 8c** shows the result of analyzing an exosome extracted from human adipose-derived mesenchymal stem cells and the result of analyzing a structural change inside the HA-PG hydrogel when the exosome is introduced into the HA-PG hydrogel patch.
**FIG. 9a** shows the physical properties and mechanical properties of a fabricated exosome hydrogel patch.
**FIG. 9b** shows the physical properties and mechanical properties of a fabricated exosome hydrogel patch.
**FIG. 10a** shows the adhesion functionality of the fabricated exosome hydrogel patch.
**FIG. 10b** shows the adhesion functionality of the fabricated exosome hydrogel patch.
**FIG. 10c** shows the adhesion functionality of the fabricated exosome hydrogel patch.
**FIG. 11a** shows the result of analyzing a chemical cross-linking mechanism of the fabricated hydrogel patch.
**FIG. 11b** shows the result of analyzing a chemical cross-linking mechanism of the fabricated hydrogel patch.
**FIG. 12a** shows the structural stability of the fabricated hydrogel patch identified by analyzing swelling and degradation patterns.
**FIG. 12b** shows the structural stability of the fabricated hydrogel patch identified by analyzing swelling and degradation patterns.
**FIG. 13a** shows the result of evaluating cytotoxicity of the fabricated hydrogel patch.
**FIG. 13b** shows the result of evaluating cytotoxicity of the fabricated hydrogel patch.
**FIG. 13c** shows the result of evaluating cytotoxicity of the fabricated hydrogel patch.
**FIG. 13d** shows the result of evaluating cytotoxicity of the fabricated hydrogel patch.
**FIG. 14a** shows the result of analyzing the exosome release behavior and activity of the fabricated hydrogel patch.
**FIG. 14b** shows the result of analyzing a exosome release behavior and activity of the fabricated hydrogel patch.
**FIG. 15a** shows the efficacy of diabetic wound treatment of the fabricated hydrogel patch.
**FIG. 15b** shows the efficacy of diabetic wound treatment of the fabricated hydrogel patch.

### BEST MODE FOR CARRYING OUT THE INVENTION

The present inventors have developed an exosome sustained topical delivery system based on a phenol group (catechol, gallol group)-functionalized hyaluronic acid derivative hydrogel patch that mimics the adhesive components of marine organisms such as mussels and sea squirts. Exosomes extracted from stem cells contain various growth factors that help tissue regeneration and anti-inflammatory factors and thus are being actively researched as therapeutic agents to substitute for stem cells, which are not easy to apply clinically. In the present disclosure, a formulation was prepared in which an exosome extracted from human adipose-derived mesenchymal stem cells is loaded in a phenol group-functionalized hyaluronic acid hydrogel patch.

In order to fabricate a hydrogel by cross-linking a hyaluronic acid polymer derivative functionalized with a phenol group, a cross-linking agent such as an oxidizing agent needs to be added. However, when an exosome is loaded, a functional group and a phenol group of protein and lipid components constituting the membrane of the exosome react to form a hydrogel without additional treatment of a cross-linking agent, which can enhance its mechanical properties compared with a hydrogel patch without the exosome. Further, it was confirmed that the physical properties and mechanical properties of the hydrogel can be controlled by controlling the concentration of the exosome.

In the present disclosure, it was confirmed that the loaded exosome directly participates in cross-linking through a reaction with the oxidized phenol group and thus can be efficiently captured inside the hydrogel, which reinforces the mechanical properties and enables sustained release of the exosome. Therefore, the exosome delivery system based on the phenol group-functionalized hyaluronic acid hydrogel patch constructed in the present disclosure is expected to show more improved tissue regeneration and therapeutic efficacy compared with simple injection of only an exosome or a conventional hydrogel-based exosome delivery technology.

The present disclosure provides an exosome hydrogel patch, including: a hydrogel patch containing a biocompatible polymer functionalized with a phenol group; and an exosome loaded in the hydrogel patch.

First, the exosome hydrogel patch according to the present disclosure includes a hydrogel patch containing a biocompatible polymer functionalized with a phenol group.

As used herein, the term "phenol group" is a functional group derived from a phenol-based compound, desirably a functional group derived from a catechol-based compound including 1,2-dihydroxybenzene having two hydroxyl groups (-OH) located adjacent to each other or a functional group derived from a pyrogallol-based compound including 1,2,3-trihydroxybenzene having three hydroxyl groups (-OH) located adjacent to each other, at the terminal. The phenol group can form covalent cross-linking with various functional groups through an oxidation reaction. Desirably, the hydrogel patch may further include a terminal functional group for reaction with the biocompatible polymer in addition to the phenol group, but is not limited thereto.

Specifically, the catechol-based compound may be selected from the group consisting of catechol, 4-tert-butylcatechol (TBC), urushiol, alizarin, dopamine, dopamine hydrochloride, 3,4-dihydroxyphenylalanine (DOPA), caffeic acid, norepinephrine, epinephrine, 3,4-dihydroxyphenylacetic acid (DOPAC), isoprenaline, isoproterenol and 3,4-dihydroxybenzoic acid. In the present disclosure, dopamine hydrochloride is used as a catechol-based compound, and in this case, -NH₂ in a terminal functional group of dopamine hydrochloride may react with the biocompatible polymer (particularly, hyaluronic acid).

Also, the pyrogallol-based compound may be selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol. In the present disclosure, 5-hydroxydopamine is used as a pyrogallol-based compound, and in this case, -NH₂ in a terminal functional group of 5-hydroxydopamine may react with the biocompatible polymer (particularly, hyaluronic acid).

In particular, a pyrogallol group used as a phenol group can be naturally oxidized within minutes without treatment of an oxidizing agent when exposed to oxygen present in the body due to its rapid oxidizing properties. Therefore, when the exosome hydrogel patch is actually applied to clinical practice, it can be directly applied without treatment of an oxidizing agent.

As used herein, the term "biocompatible polymer" may react with a terminal functional group present in the phenol-based compound so as to be functionalized with the phenol group. Specifically, the biocompatible polymer may be selected from the group consisting of hyaluronic acid, heparin, cellulose, dextran, alginate, chitosan, chitin, collagen, gelatin, chondroitin sulfate, pectin, keratin and fibrin, and may be desirably hyaluronic acid and may be more desirably hyaluronic acid having a molecular weight of from 100 kDa to 10 MDa, but is not limited thereto. In that case, -COOH in a terminal functional group of hyaluronic acid may react with the phenol-based compound.

As used herein, the term "hydrogel patch" refers to a structure formed into a thin film having a predetermined thickness and including a biocompatible polymer functionalized with a phenol group, and can be cut or molded into a desired shape by a known method and thus is convenient to use. The hydrogel patch has excellent mechanical properties, tissue adhesion, biocompatibility and ease of use compared with a solution-based bulk hydrogel.

Specifically, the hydrogel patch may be prepared by the following processes:
(a) a process of pouring a biocompatible polymer solution functionalized with a phenol group evenly on a flat surface; and
(b) a process of freeze-drying the solution at -0.5°C to -100°C for 5 hours to 48 hours.

Specifically, the process (a) may be performed by pouring 40 µl to 200 µl of a biocompatible polymer solution functionalized with a phenol group into a cylindrical mold, and the biocompatible polymer solution functionalized with a phenol group may be used at a concentration of from 0.1 (w/v)% to 5 (w/v)% and desirably from 0.5 (w/v)% to 3 (w/v)%. The amount of the biocompatible polymer solution functionalized with a phenol group is required to form a hydrogel patch having a thickness of from 0.8 mm to 3.2 mm, and the thickness of the hydrogel patch can be easily regulated.

Furthermore, the process (b) may be performed by freeze-drying the biocompatible polymer solution functionalized with a phenol group at -0.5°C to -100°C for 5 hours to 48 hours, or desirably by freeze-drying at -50°C to -100°C for 12 hours to 36 hours. When the biocompatible polymer solution functionalized with a phenol group is freeze-dried, the volume of the solution is decreased and a hydrogel patch in the form of a thin film having a predetermined thickness is formed.

As a result, the hydrogel patch may have i) a thickness of from 0.05 mm to 10.0 mm, desirably from 0.1 mm to 5.0 mm and more desirably from 1.6 mm to 5.0 mm, ii) a storage modulus G' of from 1×10³ Pa to 1×10⁶ Pa and desirably from 2×10³ Pa to 1×10⁶ Pa and tanδ of from 0.01 to 0.15 in a frequency range of from 0.1 Hz to 10 Hz, iii) a friction factor of from 0.2 to 0.4 as measured at a speed of 0.01 m/s under a normal force of 5 N, and iv) an adhesive strength of from 0.1 N to 10 N, desirably from 0.2 N to 1.6 N, more desirably from 0.25 N to 1.55 N and most desirably from 0.3 N to 0.57 N. If the hydrogel patch is a pyrogallol group-functionalized biocompatible polymer hydrogel patch, the mechanical properties thereof can be further improved.

Also, the exosome hydrogel patch according to the present disclosure includes the exosome loaded in the hydrogel patch. The amount of the exosome may be from 0.002 wt% to 10 wt% and desirably from 0.002 wt% to 4 wt% based on the total amount of the exosome hydrogel patch, but is not limited thereto. In other words, 100 ng to 2 mg of the exosome can be loaded in each hydrogel patch (based on a diameter of from 0.05 mm to 10.0 mm and a thickness of from 0.05 mm to 10.0 mm and desirably based on a diameter of from 0.1 mm to 5.0 mm and a thickness of from 0.1 mm to 5.0 mm). Specifically, the exosome hydrogel patch may be fabricated containing the exosomes at a concentration of from 1 µg/ml to 250 µg/ml, more specifically from 50 µg/ml to 100 µg/ml and more specifically 50 µg/ml or 100 µg/ml when the above-described hydrogel patch is fabricated.

It can be effectively released in a sustained manner in vivo and thus can induce a lasting therapeutic effect. Various nucleophilic functional groups may exist in the exosome and may form a strong bond with the oxidized phenol group.

Specifically, the nucleophilic functional groups (amine group, thiol group, imidazole group, etc.) present in the exosome can form a strong bond with the oxidized phenol group and thus can be effectively released in a sustained manner in vivo.

Specifically, a method of loading the exosome in the hydrogel patch may be performed by mixing a biocompatible polymer solution functionalized with a phenol group and an exosome to fabricate a hydrogel patch, or by applying an exosome onto a phenol group-functionalized biocompatible polymer hydrogel patch and then cross-linking the exosome to the phenol group-functionalized biocompatible polymer hydrogel patch through treatment of an oxidizing agent. In this case, the treatment of the oxidizing agent may be performed by applying or spraying an oxidizing agent solution to an exosome-applied hydrogel patch. However, if the hydrogel patch is functionalized with a pyrogallol group, it is naturally oxidized in vivo without treatment of an oxidizing agent and thus is easy to use in actual clinical practice.

### MODE FOR CARRYING OUT THE INVENTION

Hereafter, one or more specific examples will be described in more detail. However, these examples are provided for illustrative purposes only and the scope of the present disclosure is not limited to these examples.

### Preparation Example 1

### (1) Fabrication of HA-CA hydrogel patch

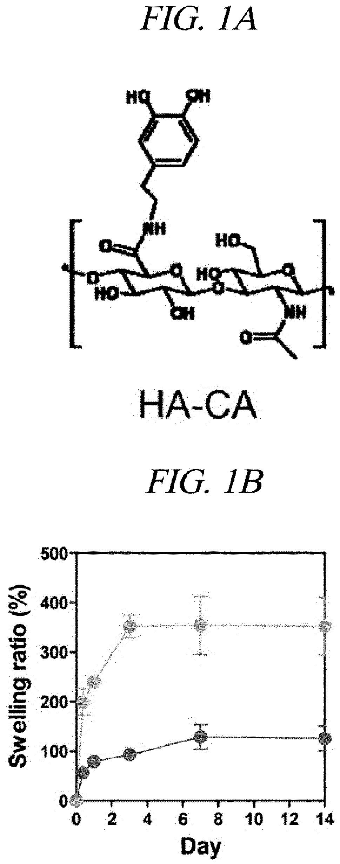

A catechol-functionalized hyaluronic acid with dopamine hydrochloride (hereinafter, referred to as HA-CA, **FIG. 1A**) (molecular weight=200 kDa) was dissolved in distilled water at a concentration of 1 (w/v)%, and 80 µl of the 1 (w/v)% HA-CA solution was poured into an 8 mm cylindrical mold and then freeze-dried overnight at -80°C to fabricate an HA-CA hydrogel patch with a diameter of 8 mm and a thickness of 1.6 mm. The fabricated HA-CA hydrogel patch is in a dry state and thus is easy to store. Also, it is fabricated in the form of a thin film and thus can be easily cut into a desired shape. Therefore, it is convenient to use.

Meanwhile, HA-CA was dissolved in phosphate-buffered saline (PBS), and a 4.5 mg/ml sodium periodate solution was added into the solution to fabricate an HA-CA bulk hydrogel. A final concentration of HA-CA in the fabricated HA-CA bulk hydrogel was 1 (w/v)%.

### (2) Analysis on physical properties of HA-CA hydrogel patch

The HA-CA hydrogel patch or the HA-CA bulk hydrogel was immersed in PBS at 37°C similar to in vivo conditions for 14 days, and the swelling ratio was measured after 12 hours, 1 day, 3 days, 7 days and 14 days. As a result of measurement, the swelling ratio of the HA-CA hydrogel patch (Patch) was higher than that of the HA-CA bulk hydrogel (Gel) (**FIG. 1B**)**.**

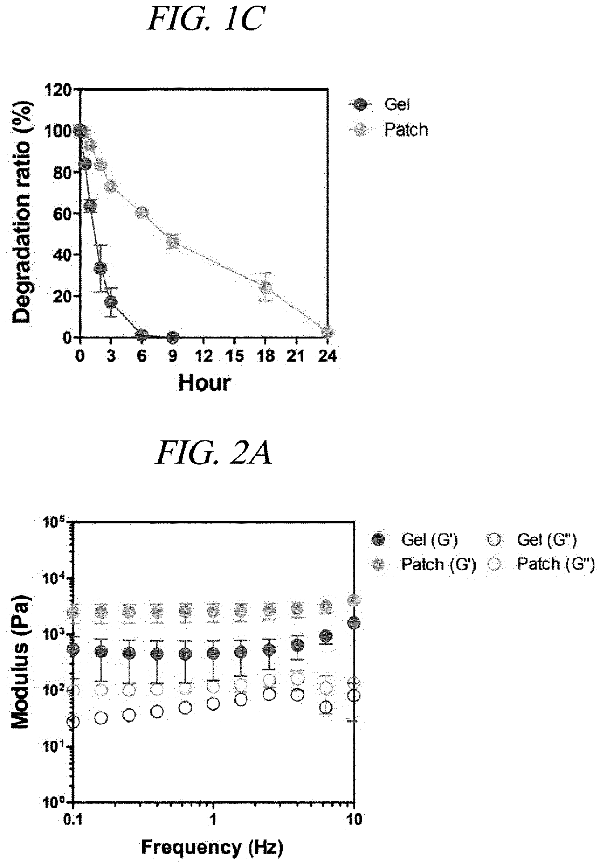

Since various degradation enzymes exist in the actual in vivo environment, the HA-CA hydrogel patch or the HA-CA bulk hydrogel was immersed in PBS at 37°C and then treated with a hyaluronidase until degradation (100 U/sample). The weight of the HA-CA hydrogel patch or the HA-CA bulk hydrogel was measured at regular time intervals to measure the degrees of degradation over time. As a result of measurement, the HA-CA bulk hydrogel (Gel) was rapidly degraded within 2 hours after the treatment with the hyaluronidase and completely degraded after 6 hours. However, the HA-CA hydrogel patch (Patch) remained even after 24 hours from hyaluronidase treatment, thus indicating a decreased enzymatic degradation rate (**FIG. 1C**).

### (3) Analysis on mechanical properties of HA-CA hydrogel patch

The elastic modulus of the HA-CA hydrogel patch or the HA-CA bulk hydrogel was measured at a frequency of from 0.1 Hz and 10 Hz by using a rheometer. As a result of analysis, both the HA-CA hydrogel patch (Patch) and the HA-CA bulk hydrogel (Gel) showed higher storage moduli G' than loss moduli G". Thus, it was found that the internal structure is formed of a stable polymer network (**FIG. 2A**).

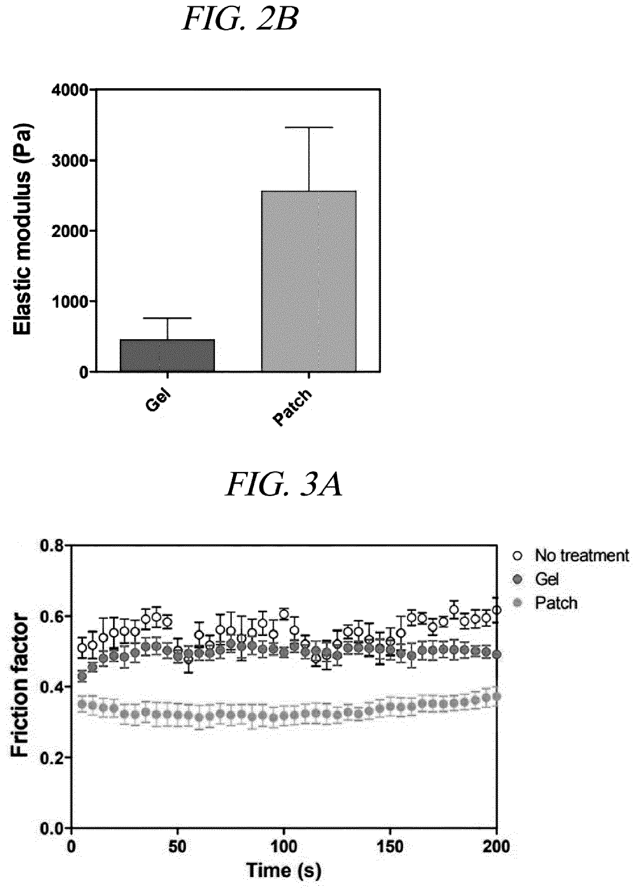

Further, the average storage modulus G' of the HA-CA bulk hydrogel (Gel) was about 450 Pa, whereas the average storage modulus G' of the HA-CA hydrogel patch (Patch) was in the range of from about 2500 Pa to about 2600 Pa. Thus, it was found that the average storage modulus G' increased by more than five-fold (**FIG. 2B**).

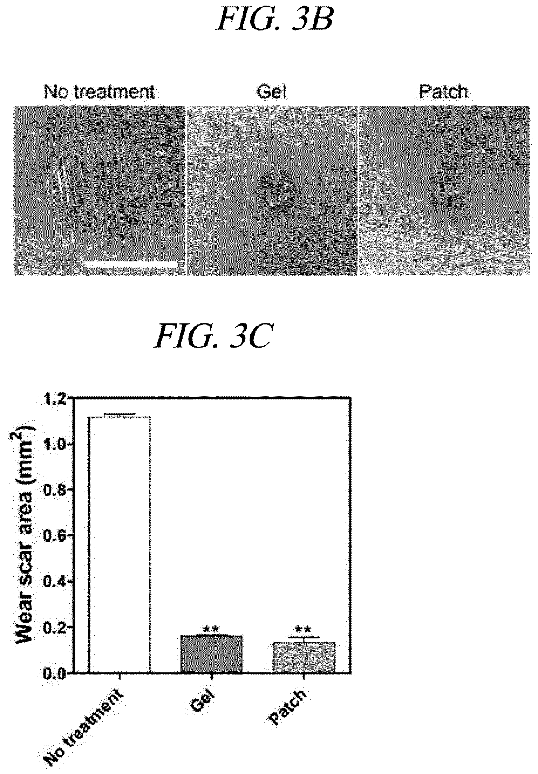

Meanwhile, a friction factor was measured by moving a friction force analyzer at a speed of 0.01 m/s in a state where a normal force of 5 N was applied between stainless steel surfaces coated with the HA-CA hydrogel patch or the HA-CA bulk hydrogel. As a result of measurement, the friction factor was the highest in the case of no coating (No treatment), followed by the HA-CA bulk hydrogel (Gel) and the HA-CA hydrogel patch (Patch) (**FIG. 3A**). Further, in the case of no coating (No treatment), a large wear scar was formed due to friction, and when the HA-CA bulk hydrogel (Gel) and the HA-CA hydrogel patch (Patch) were coated, a small wear scar was formed (**FIG. 3B**). Therefore, when the HA-CA bulk hydrogel (Gel) and the HA-CA hydrogel patch (Patch) were coated, the area of wear scar was significantly reduced compared with the case of no coating (No treatment) (**FIG. 3C**).

### Preparation Example 2

### (1) Fabrication of HA-PG hydrogel patch

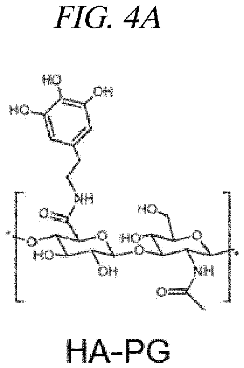

A pyrogallol-functionalized hyaluronic acid with 5-hydroxydopamine (hereinafter, referred to as HA-PG, **FIG. 4A**) (molecular weight=200 kDa and 1 MDa) was dissolved in distilled water at a concentration of 1 (w/v)%, and 80 µl of the 1 (w/v)% HA-PG solution was poured into an 8 mm cylindrical mold and then freeze-dried overnight at -80°C to fabricate an HA-PG hydrogel patch with a diameter of 8 mm and a thickness of 1.6 mm. The fabricated HA-PG hydrogel patch is in a dry state and thus is easy to store. Also, it is fabricated in the form of a thin film and thus can be easily cut into a desired shape. Therefore, it is convenient to use.

Meanwhile, HA-PG was dissolved in phosphate-buffered saline (PBS), and a 4.5 mg/ml sodium periodate solution was added into the solution to fabricate an HA-PG bulk hydrogel. A final concentration of HA-PG in the fabricated HA-PG bulk hydrogel was 1 (w/v)%.

### (2) Analysis on physical properties of HA-PG hydrogel patch

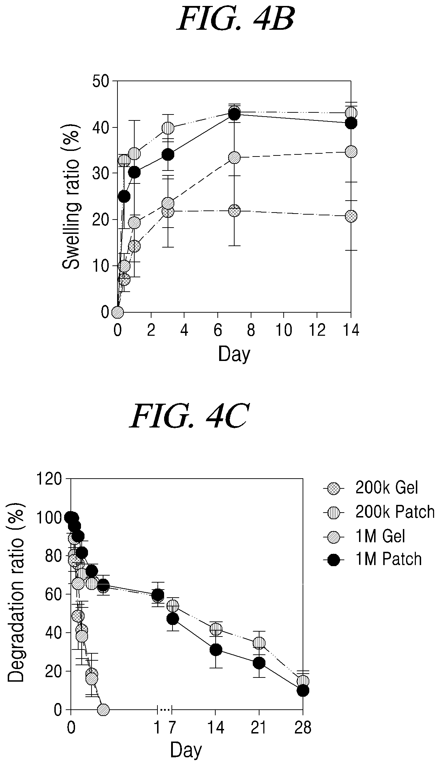

The HA-PG hydrogel patch or the HA-PG bulk hydrogel was immersed in PBS at 37°C similar to in vivo conditions for 14 days, and the swelling ratio was measured after 12 hours, 1 day, 3 days, 7 days and 14 days. As a result of measurement, the swelling ratio of the HA-PG hydrogel patch (Patch) was higher than that of the HA-PG bulk hydrogel (Gel) (**FIG. 4B**).

Since various degradation enzymes exist in the actual in vivo environment, the HA-PG hydrogel patch or the HA-PG bulk hydrogel was immersed in PBS at 37°C and then treated with a hyaluronidase until degradation (200 U/sample). The weight of the HA-PG hydrogel patch or the HA-PG bulk hydrogel was measured at regular time intervals to measure the degrees of degradation over time. As a result of measurement, the HA-PG bulk hydrogels (200 kDa and 1 MDa Gels) were rapidly degraded at an early stage after the treatment with the hyaluronidase. However, the HA-PG hydrogel patches (200 kDa and 1 MDa Patches) remained even after 28 days from hyaluronidase treatment, thus indicating a considerably decreased enzymatic degradation rate. (**FIG. 4C**).

### (3) Analysis on mechanical properties of HA-PG hydrogel patch

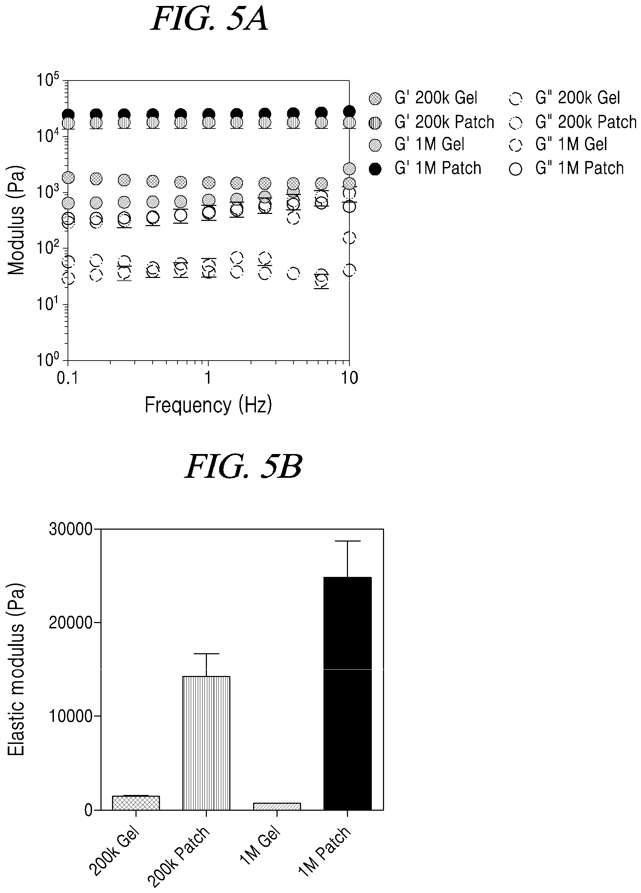

The elastic modulus of the HA-PG hydrogel patch or the HA-PG bulk hydrogel was measured at a frequency of from 0.1 Hz and 10 Hz by using a rheometer. As a result of analysis, both the HA-PG hydrogel patch (Patch) and the HA-PG bulk hydrogel (Gel) showed higher storage moduli G' than loss moduli G". Thus, it was found that the internal structure is formed of a stable polymer network (**FIG. 5A**).

Further, the average storage moduli G' of the HA-PG bulk hydrogels (200 kDa and 1 MDa Gels) were very low, whereas the average storage moduli G' of the HA-PG hydrogel patches (200 kDa and 1 MDa Patches) were in the range of from about 14 kPa to about 24 kPa. Thus, it was found that the average storage moduli G' increased considerably (**FIG. 5B**).

Meanwhile, a friction factor was measured by moving a friction force analyzer at a speed of 0.01 m/s in a state where a normal force of 5 N was applied between stainless steel surfaces coated with the HA-PG hydrogel patch or the HA-PG bulk hydrogel. As a result of measurement, the friction factor was the highest in the case of no coating (No treatment), followed by the HA-PG bulk hydrogels (200 kDa and 1 MDa Gels) and the HA-PG hydrogel patches (200 kDa and 1 MDa Patches)

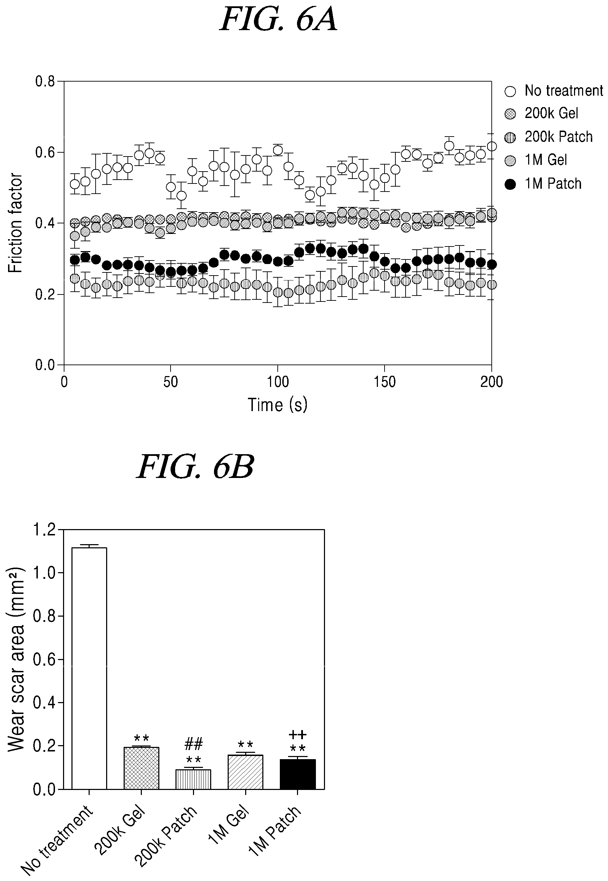

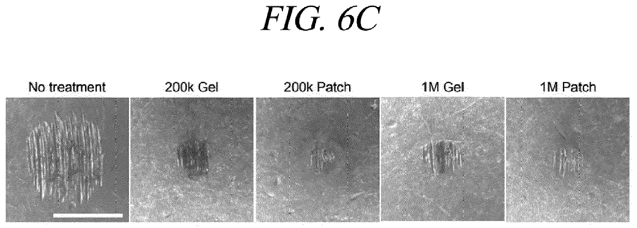

(**FIG. 6A**). Further, in the case of no coating (No treatment), a large wear scar was formed due to friction, and when the HA-PG bulk hydrogels (200 kDa and 1 MDa Gels) and the HA-PG hydrogel patches (200 kDa and 1 MDa Patches) were coated, a small wear scar was formed (**FIG. 6B**). Therefore, when the HA-PG bulk hydrogels (200 kDa and 1 MDa Gels) and the HA-PG hydrogel patches (200 kDa and 1 MDa Patches) were coated, the area of wear scar was significantly reduced compared with the case of no coating (No treatment) (**FIG. 6C**).

### Example 1: Characterization of exosome-loaded HA-PG hydrogel

### 1-1. Fabrication of exosome-loaded HA-PG hydrogel (exosome hydrogel patch)

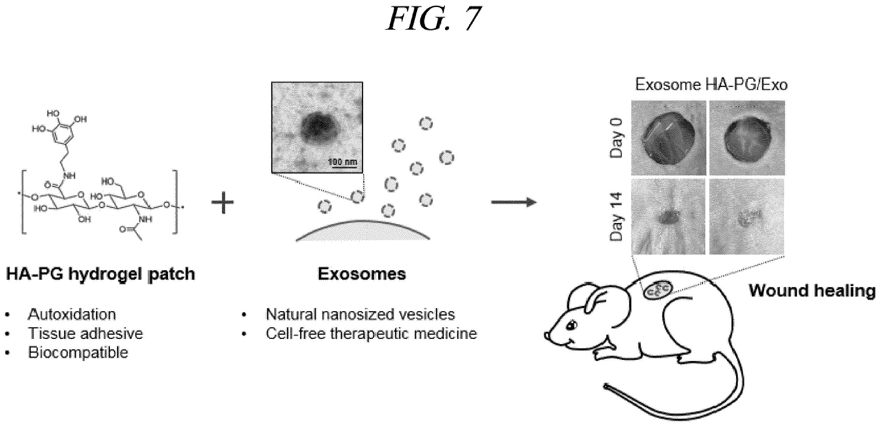

In the present disclosure, a HA-PG derivative was synthesized by introducing a gallol group (PG) into hyaluronic acid (HA), a natural polymer, and mixed with an exosome extracted from human adipose-derived mesenchymal stem cells in an HA-PG solution, followed by freeze-drying to fabricate an exosome-loaded patch formulation (**FIG. 7**).

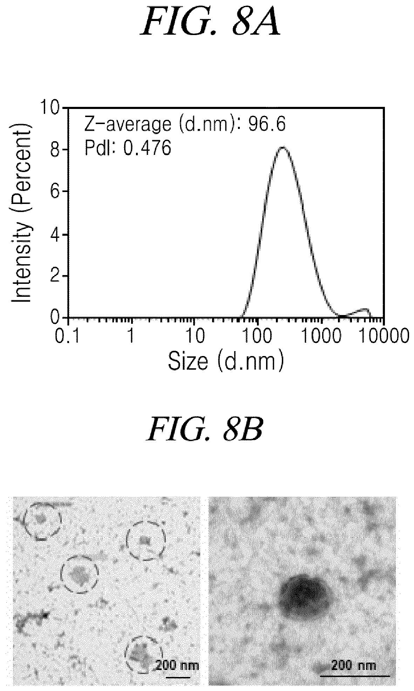

As a result of check on a size distribution pattern of the exosome extracted from human adipose-derived mesenchymal stem cells through DLS analysis, it was observed that the exosome had an average diameter of 96.6 nm and a uniform size with a Pdl value of 0.476 (**FIG. 8A**). Also, the shape and size of the exosome was observed again by transmission electron microscopy (TEM) analysis, where it was found that the exosome had a spherical nanoparticle shape with a size of 94.17 nm, which confirmed agreement with the DLS analysis result (**FIG. 8B**).

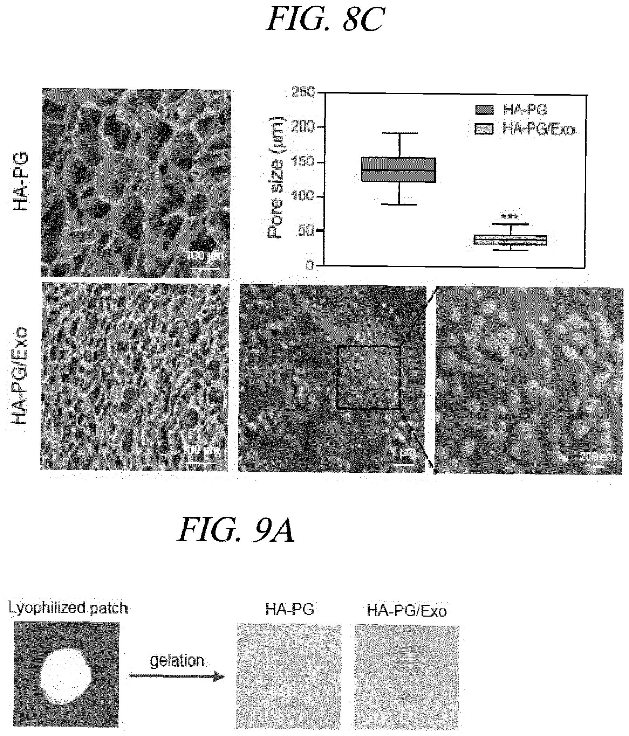

When the internal structure of the patch fabricated by preparing a 1% (w/v) HA-PG solution in which an exosome is mixed with an HA-PG solution and freeze-drying the 1% (w/v) HA-PG solution was examined by scanning electron microscopy (SEM) analysis, It was found that the exosome-loaded HA-PG patch had a denser porous structure and exosome particles with a diameter of 100 nm were present on the surface of the internal structure (**FIG. 8C**).

### 1-2. Check on physical properties of fabricated exosome hydrogel patch

The HA-PG derivative can originally be cross-linked through natural oxidation of the gallol group over time, and can be cross-linked faster and more stably due to the introduction of the exosome. Due to a high reactivity between the functional groups (amine group, thiol group, imidazole group, etc.) of various proteins included in the exosome and the oxidized gallol group, the loaded exosome can further promote cross-linking inside the HA-PG hydrogel.

Specifically, the HA-PG hydrogel becomes deeper yellow as oxidation proceeds. After gelation was induced in an exosome-loaded patch and a patch without an exosome at 37°C under PBS conditions for the same period of time, the degree of cross-linking was determined by a difference in color (**FIG. 9A**).

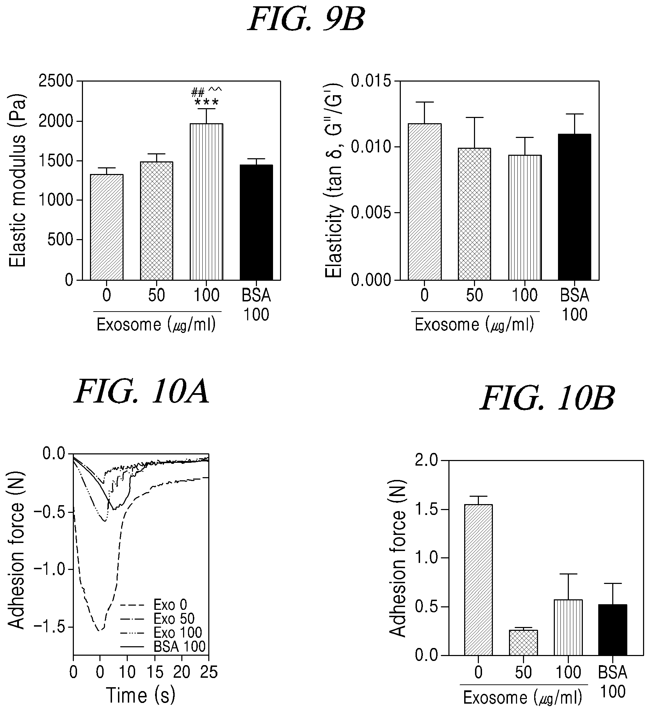

It was confirmed through rheometer analysis (frequency sweep mode) that as the concentration of the exosome increased, the physical properties of the hydrogel patch were improved and the elasticity was maintained (**FIG. 9B**, Exo 0 group; 1% HA-PG patch, Exo 50 group; 1% HA-PG patch loaded with 50 µg/ml exosome, Exo 100 group; 1% HA-PG patch loaded with 100 µg/ml exosome, BSA 100 group; 1% HA-PG patch loaded with 100 µg/ml BSA). The modulus of the exosome patch (100 group) was measured higher than that of the patch (BSA 100 group) added with BSA protein at the same concentration as the exosome. Therefore, it can be seen that the exosome can induce improvements in physical properties of the HA-PG hydrogel patch (**FIG. 9B**).

### 1-3. Check on adhesion functionality of fabricated exosome hydrogel patch

After cross-linking a substrate for tack test and the hydrogel patch by natural oxidation, the adhesion was checked (**FIG. 10A**)

It was found that the HA-PG patch loaded with 50 µg/ml exosome (Exo 50 group) decreased in adhesion compared with the HA-PG patch without an exosome (Exo 0 group), but the HA-PG patch with an increased concentration of the exosome (Exo 100 group) restored the adhesion up to a level equivalent to 36.8% of the adhesion of the patch without an exosome (**FIG. 10B**, Exo 0 group; 1.55 N, Exo 100 group; 0.57 N).

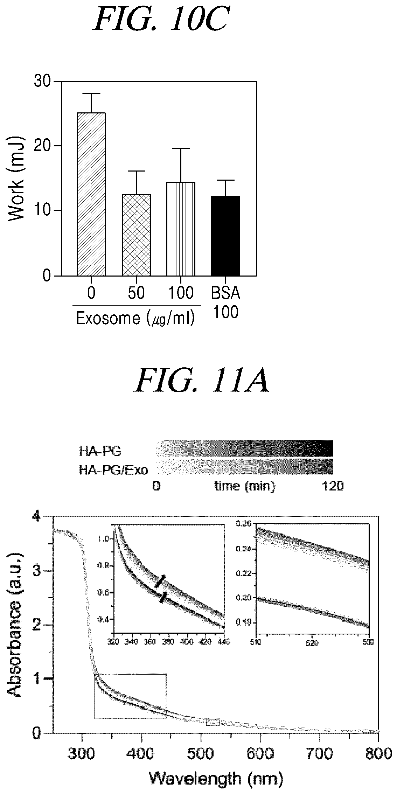

Furthermore, a similar tendency was found when the energy required for the hydrogel patch to be separated from the substrate for tack test was calculated by integrating the adhesion graph (**FIG. 10C**). In particular, the energy required for the hydrogel patch of Exo 100 group to be separated from the substrate for tack test substrate was 57.9% of the hydrogel patch of Exo 0 group, which confirmed that even if the exosome is loaded in the HA-PG patch, the adhesion of the patch can be maintained at a substantial level (**FIG**. **10C****,** Exo 0 group; 25.1 mJ, Exo 100 group; 12.4 mJ).

### 1-4. Analysis of chemical cross-linking mechanism of fabricated exosome hydrogel patch

Functional groups (amine, thiol, imidazole, etc.) of various proteins and lipids present in the exosome are expected to react with the gallol group and increase the cross-linking rate of the HA-PG hydrogel, which was confirmed.

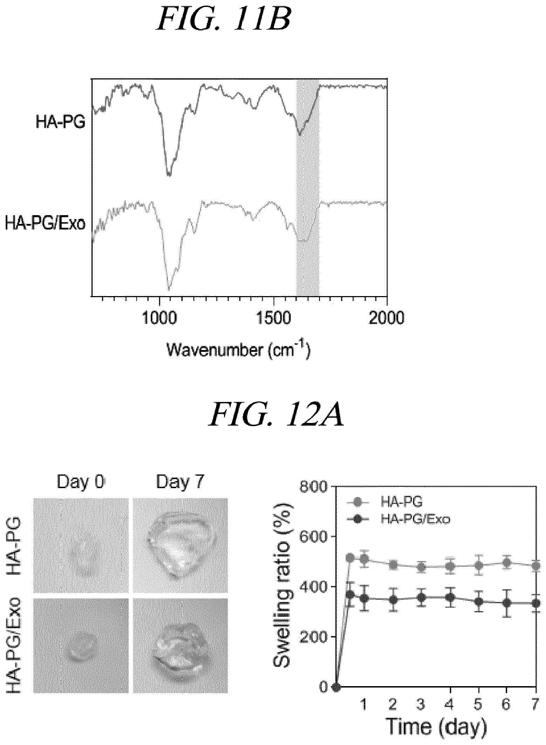

Specifically, for analysis of the chemical cross-linking mechanism of the exosome patch, a 2% HA-PG solution (HA-PG group) and an HA-PG solution containing 200 µg/ml exosome (HA-PG/Exo group) were naturally oxidized (37°C incubation), followed by UV-vis spectroscopy analysis. As a result, it was found that a peak in the range of from 300 nm to 450 nm increased faster in the HA-PG/Exo group than in the HA-PG group (**FIG. 11** **A**). Also, the peak is a broad peak formed by semi-quinone intermediate (corresponding to a peak at from 350 nm to 380 nm), phenoxyl radical (corresponding to a peak at from 350 nm to 380 nm), and purpurogallin (corresponding to peaks at 325 nm and from 420 nm to 440 nm) caused by natural oxidation of the HA-PG derivative. Therefore, it was confirmed that the addition of the exosome increased the rate of natural oxidation of the HA-PG derivative. Further, it was found that a peak in the range of from 510 nm to 530 nm specifically increased only in the HA-PG/Exo group. The peak can be assumed to change due to the interaction between the gallol group and the lipid surface of the exosome.

Furthermore, different spectra at the amide I & II peaks around 1500 cm⁻¹ to 1700 cm⁻¹ found by comparison between HA-PG and HA-PG/Exo through FT-IR analysis confirmed that there is an interaction between the exosome and HA-PG (**FIG. 11B**).

### 1-5. Check on structural stability of fabricated exosome hydrogel patch (analysis of swelling and degradation patterns)

The swelling and degradation patterns of the exosome hydrogel patch (1% HA-PG patch loaded with 100 µg/ml exosome) were compared with those of the HA-PG patch without an exosome (1% HA-PG patch).

Under physiological conditions (37°C, PBS), the exosome HA-PG patch showed a lower swelling ratio than the HA-PG patch (**FIG. 12A**), which indirectly confirms that the exosome-loaded HA-PG hydrogel patch has a denser internal structure than the HA-PG patch.

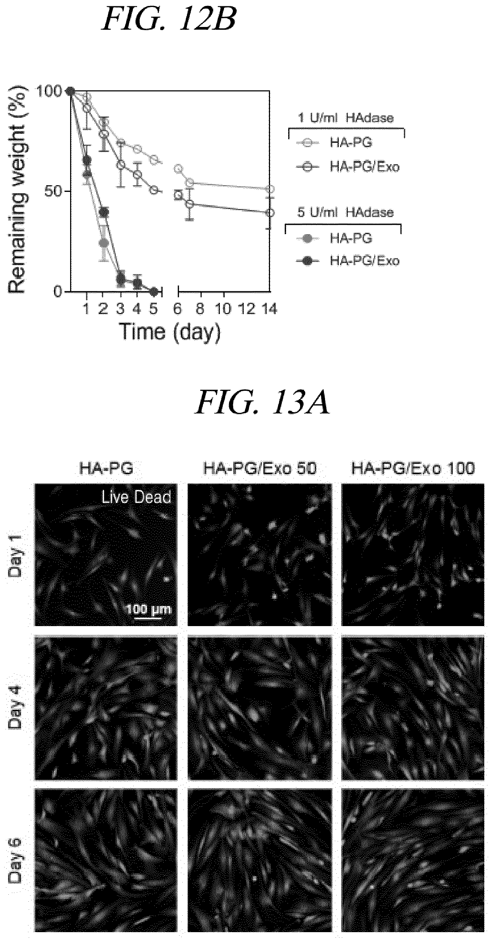

When the exosome HA-PG patch and the HA-PG patch were treated with the same concentration (activity) of a hyaluronidase (HAdase), there was no significant difference in degradation pattern at a high concentration (5 U/ml HAdase), but the exosome hydrogel patch degraded somewhat faster than the HA-PG patch at a low concentration (1 U/ml HAdase) (**FIG. 12B**)**.**

### 1-6. Evaluation of cytotoxicity of fabricated exosome hydrogel patch

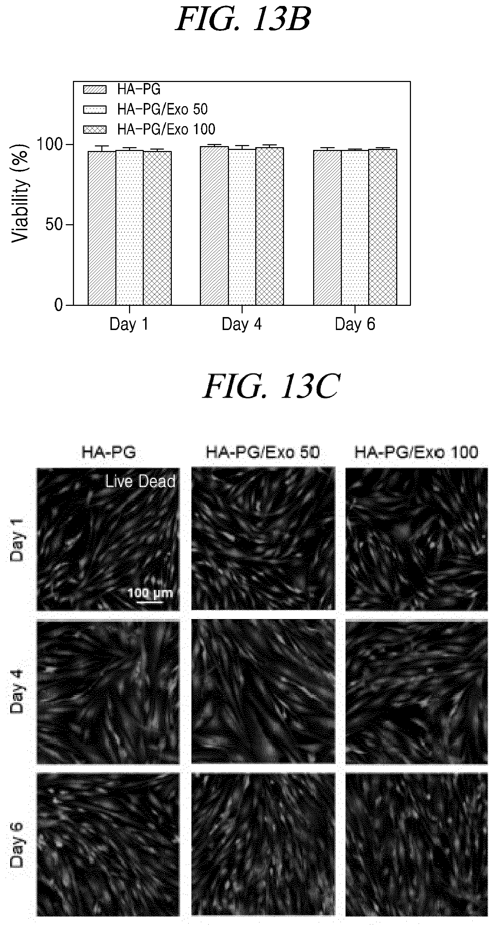

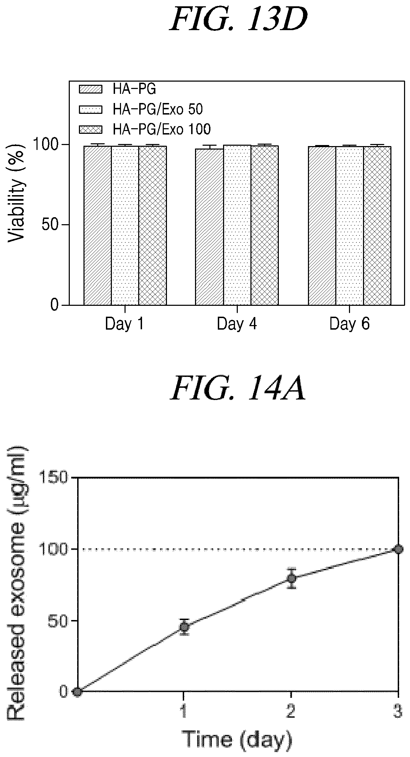

The HA-PG patch (1% HA-PG patch) or the exosome HA-PG patch (1% HA-PG patch loaded with 50 or 100 µg/ml exosome) was incubated for 24 hours under physiological conditions (37°C, cell culture medium) and then, the cell culture medium was collected and used for treating and incubating human adipose-derived mesenchymal stem cells **(****FIGS. 13A** and **13B****)** and human fibroblasts **(****FIGS. 13C** and **13D****)** to evaluate the cytotoxicity thereof (**FIG. 13**).

As a result of checking the cell viability by Live/Dead staining during incubation for 6 days, it was found that all groups showed excellent cell viability, which confirmed that both the HA-PG patch and the exosome HA-PG patch had excellent biocompatibility.

### 1-7. Analysis of exosome release behavior and activity of fabricated exosome hydrogel patch

To evaluate whether the exosome HA-PG hydrogel patch (1% HA-PG patch loaded with 100 µg/ml exosome) can be used as a sustained release formulation of exosome, the amount of exosome released was checked through BCA assay during a treatment with a hyaluronidase (1 U/ml HAdase) present in the human body (**FIG. 14A**)**.** It was confirmed from this result that the exosome hydrogel patch can release the exosome in a sustained manner over 3 days. Therefore, it can be seen that the therapeutic effect can be achieved through effective exosome delivery into a tissue defect site, such as a wound site, where the activity of the hyaluronidase is increased.

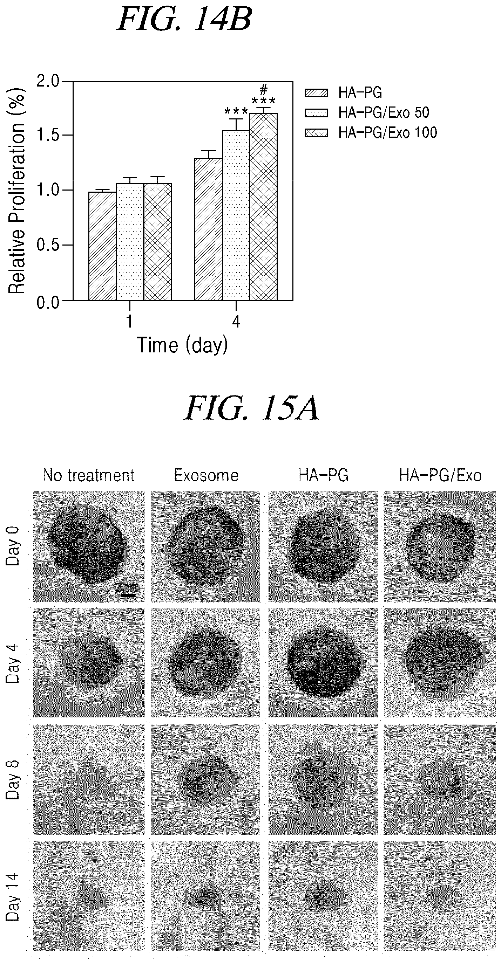

When human fibroblasts were three-dimensionally incubated in the hydrogel patch and after 4 days, a cell proliferation pattern was compared through MTT analysis, the proliferation of cells incubated in the exosome hydrogel patch was promoted by 1.5 to 1.7 times or more compared with that of a sample incubated in the HA-PG hydrogel patch for one day (**FIG. 14B**). It can be seen that the exosome released from the exosome hydrogel patch is effective for the survival and proliferation of cells, and, thus, the exosome hydrogel patch can be used as a sustained release formulation for tissue regeneration in disease sites.

### Example 2: Check on diabetic wound therapeutic efficacy of fabricated exosome hydrogel patch

The possibility of using the exosome patch as a therapeutic agent for tissue regeneration was checked through animal model experiments.

Specifically, mice were administered intraperitoneally twice (24-hour interval) with 0.1 g/kg streptozotocin to induce diabetes. After 14 days, fasting blood glucose was measured and only mice with a blood glucose level of 300 md/dL or more were used as diabetic wound models. After inducing a circular wound with a diameter of 8 mm in the back skin of each mouse using a biopsy punch, the wound was treated with a hydrogel patch and then, any improvement in tissue regeneration through sustained release of an exosome was checked (**FIG. 15A**).

The exosome hydrogel patch (1% HA-PG patch loaded with 100 µg/ml exosome) was able to be stably attached to the wound site based on its adhesion functionality without a separate cross-linking agent, and also able to protect the wound site from external stimuli and induce effective exosome delivery due to its improved physical properties compared with a conventional HA-PG patch.

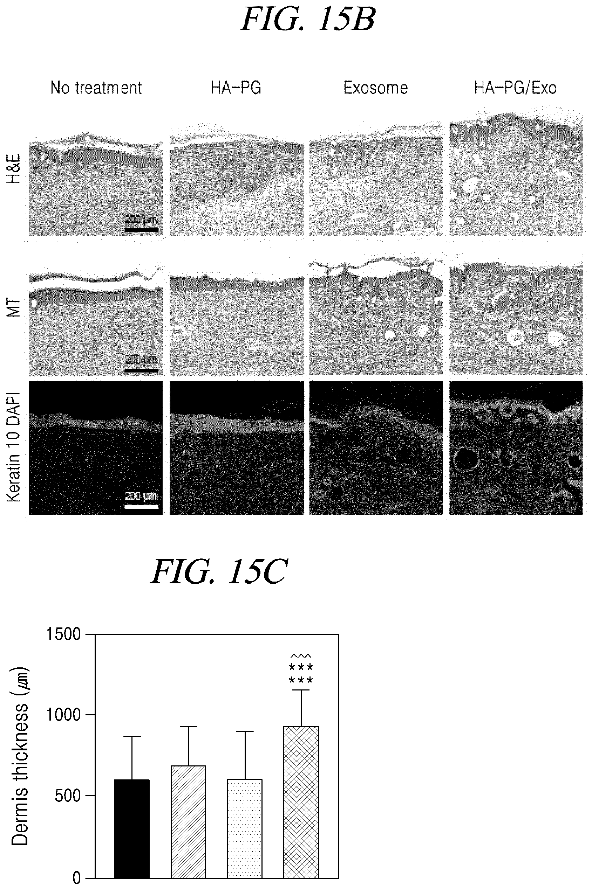

The size of the wound observed with the naked eye until 14 days after application of the patch did not show a significant difference by group (**FIG. 15A**), but when the regenerated tissue was collected and histologically analyzed, enhanced tissue regeneration by the exosome patch was confirmed (**FIGS. 15B** to **15F**)**.**

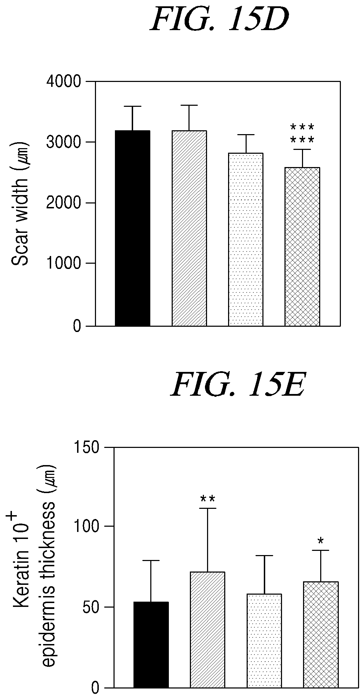

As a result of Hematoxylin & Eosin (H&E) staining and Masson's trichrome (MT) staining, it was found that when the exosome patch was applied, the newly regenerated dermal layer was the thickest (**FIG. 15C**) and the size of the abnormal wound tissue was the smallest (**FIG. 15D**). Thus, it was confirmed that the exosome patch induced effective skin regeneration.

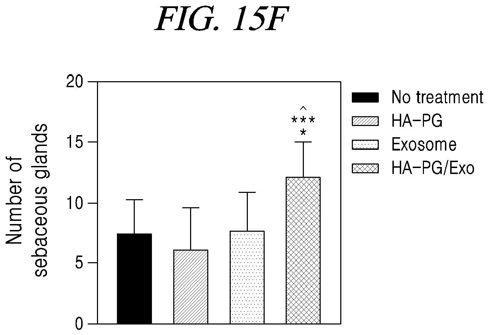

Also, it was found that in the exosome patch group, the thickness of the epidermal layer with keratin 10⁺ was significantly increased (**FIG. 15E**), and the number of skin organelles such as sebaceous glands increased the most (**FIG. 15F**). Thus, it was confirmed that sustained release of the exosome induced by using the exosome patch can maximize functional tissue regeneration of damaged skin.

The present disclosure has been described with reference to the preferred exemplary embodiments thereof. It can be understood by a person with ordinary skill in the art that the present disclosure can be implemented as being modified and changed within the scope departing from the spirit and the scope of the present disclosure. Accordingly, the above-described exemplary embodiments should be considered in descriptive sense only and not for purposes of limitation. Also, the technical scope of the present disclosure is defined not by the detailed description of the invention but by the appended claims, and all differences within the scope will be construed as being comprised in the present disclosure.

## Claims

1. An exosome hydrogel patch, comprising:
a hydrogel patch containing a biocompatible polymer functionalized with a phenol group; and
an exosome loaded in the hydrogel patch.

2. The exosome hydrogel patch of Claim 1,
wherein the phenol group is a catechol group derived from a catechol-based compound selected from the group consisting of catechol, 4-tert-butylcatechol (TBC), urushiol, alizarin, dopamine, dopamine hydrochloride, 3,4-dihydroxyphenylalanine (DOPA), caffeic acid, norepinephrine, epinephrine, 3,4-dihydroxyphenylacetic acid (DOPAC), isoprenaline, isoproterenol and 3,4-dihydroxybenzoic acid; or a pyrogallol group derived from a pyrogallol-based compound selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol.

3. The exosome hydrogel patch of Claim 1,
wherein the biocompatible polymer is selected from the group consisting of hyaluronic acid, heparin, cellulose, dextran, alginate, chitosan, chitin, collagen, gelatin, chondroitin sulfate, pectin, keratin and fibrin.

4. The exosome hydrogel patch of Claim 1,
wherein the exosome hydrogel patch has
i) a thickness of from 0.05 mm to 10.0 mm,
ii) a storage modulus G' of from 1×10³ Pa to 1×10⁶ Pa and tanδ of from 0.01 to 0.15 in a frequency range of from 0.1 Hz to 10 Hz,
iii) a friction factor of from 0.2 to 0.4 as measured at a speed of 0.01 m/s under a normal force of 5 N, and
iv) an adhesive strength of from 0.1 N to 10 N.

5. The exosome hydrogel patch of Claim 1,
wherein the exosome is derived from stem cells.
